# EUROPEAN PATENT SPECIFICATION

(11) **EP 2 501 343 B1**
(45) Date of publication and mention of the grant of the patent: **26.08.2015**
(21) Application number: 10836830.9
(22) Date of filing: 18.11.2010
(51) Int. Cl.: A61F 2/50, A61F 2/64, A61F 2/66, A61F 2/68, A61F 2/70, A61F 2/76

(54) **PROSTHESIS STRUCTURE FOR LOWER-LIMB AMPUTEES**
PROTHESENSTRUKTUR FÜR UNTERSCHENKELAMPUTIERTE
STRUCTURE DE PROTHÈSE POUR AMPUTÉS D'UN MEMBRE INFÉRIEUR

(30) Priority: 18.11.2009 IT PI20090144
(43) Date of publication of application: 26.09.2012
(73) Proprietor: Rizzoli Ortopedia S.p.A., 40054 Budrio (IT)
(72) Inventor: BALLI, Leonardo, I-50143 Firenze (FI) (IT); DONATI, Gabriele, I-51017 Pescia (PT) (IT); FERRINI, Nicola, I-56030 Terricciola (PI) (IT); GIULIANI, Pierandrea, I-00135 Roma (IT); PALLANTI, Marco, I-56021 Cascina (PI) (IT); PARRINI, Gianluca, I-56121 Cascina (PI) (IT)
(74) Representative: Celestino, Marco
(86) International application number: PCT/IB2010/002957
(87) International publication number: WO 2011/080556

(56) References cited:
- WO-A1-2007/016408
- WO-A1-2008/031220
- DE-A1-102005 020 188
- US-A1- 2006 249 315
- US-A1- 2007 156 252
- US-A1- 2009 265 018

## Description

### Field of the invention

The present invention relates to the field of orthopaedic appliances and more precisely it relates to an automatic prosthesis for lower-limb amputees. In particular, the invention relates to an ankle/foot prosthesis that can comprise also a knee portion in addition to an ankle/foot portion in case of above-knee amputees.

Furthermore, the invention relates to an electronic control apparatus capable to control this prosthesis using specific program means.

### Description of the technical problem

Various types are known of prosthesis for lower-limb amputees.
said prosthesis consist of two groups:
- The "foot", which is formed by a plurality of components and is adapted to reproduce the dorsal, plantar behaviour and the front inversion/eversion of the foot, in particular with reproduction of the kinematic rotational behaviour of the phalanxes with respect to the metatarsus of a non-disabled person;
- The "ankle", formed by the tibial segment and by a stiff element of connection of the tibial segment to the "tarsus" of the "foot"; such two elements can carry out with respect to each other a rotational movement, in particular said elements can normally carry out a purely rotational movement like a simple hinge.

In case of above-knee amputees, there is a further group:
- The "knee", formed by a femoral segment and a tibial segment connected to each other through a kinematic system with one degree of freedom; the two elements can carry out with respect to each other a rotational movement, like a simple hinge, or rototranslational, for example a polycentric system with four-bar linkages or multilink systems.

Concerning the prosthesis of the knee, where a configuration is provided with a femoral segment and a tibial segment pivotally connected to each other about an articulation axis that reproduces the movement of the knee, a hydraulic damper can be provided that connects the femoral segment with the tibial segment. An example of these prostheses is disclosed in JP52047638, GB826314, US4212087, US3599245.

Concerning the ankle/foot prosthesis, in particular prostheses are to be considered with: (1) stiff articulation, (2) mono axial articulation and (3) pluri-axial articulation of the ankle.

In case of stiff articulation there is not possibility of adjusting the position of the foot toe with respect to the tibial segment; in the mono axial articulation the possibility exists of rotating in the sagittal plane the foot toe in rear flexion or plantar flexion; finally in case of pluri-axial articulation the possibility exists of having beyond the rear flexion and the plantar flexion also an inversion/eversion in the front plane.

In addition to the above cited prostheses with purely passive ankle, also trans-tibial prostheses exist that can be electronically controlled by microprocessors and fed by suitable batteries. Said prostheses allow raising the foot toe in plane paths as well as in rising or descending paths or in static posture, ensuring an improvement of the "bio mimetic" behaviour of the prosthesis.

US 2007156252A1 is considered to represent the closest prior art, it discloses a prosthesis of the ankle with an actuator that controls and adjusts actively the angle set between a foot unit and a tibial unit. The actuator can block the angle in determined portions of the gait cycle and minimize the mechanical backlash. A sensor module contains data on the gait of the user and can be used for giving to actuator such data for reproducing the movement of the ankle of a healthy user in various situations, such as walking on a plane path, going up the stairs, walking on sloped surfaces.

One of the many problems of the existing prostheses for above-knee amputees is stumbling over the tip of the foot, so-called Toe Clearance. In particular, with a low walking speed there is a minimum dynamic effect of the femur that causes a small lifting effect of the prosthetic foot. The stiffness of the foot same, does not ensure a sufficient extension between femur and tibia during the swing gait phase, such that the patient stumbles over the tip of the foot.

Another problem, during the gait in plane paths in elder patients or in phase of rehabilitating the walking activity, further to the above-knee amputation, is realigning the tibia with the femur. In fact, once passed the Top Dead Center between the femoral segment and the tibial segment, it is difficult to achieve realignment between the femoral segment and the tibial segment owing to a minimum swinging of the tibial segment.

A further problem is the impossibility, in existing prostheses, of adjusting the speed of gait in a gait cycle. Such need is felt in situations such as unexpected obstacles, with need of changing speed for passing it, or the need of stopping quickly the gait.

Yet another problem is the difficulty, for existing prostheses, of adjusting progressively the gait parameters as the patient becomes trained with the prosthesis. Normally, it is necessary to change prosthesis or to carry out mechanical adjustments by technical experts.

Further problems are the range of the batteries of the prosthesis, requiring suitable batteries for actuating electric motors or actuators, where present, as well as an easy replacement of the batteries.

Yet another problem is that a passive prosthesis cannot adjust the pushing power of the foot, as well as the control of entering the stance phase, with the disadvantage that the impact of the foot on the ground is normally more violent that a physiological gait, and this condition generates the possibility of developing problems of orthopaedic nature to the back and to increase the average metabolic consumption with respect to a non-disabled person since the patient attempts to compensate mechanical losses using the muscles of the pelvis and of the femur.

Yet another problem is the difficulty to mimic the posture of a non-disabled person, both during the gait cycle, and during static posture such as when sitting, because in such cases a stiff foot does not allow to have a natural posture generating sometimes embarrassment to the user.

Yet another problem is the design of the prosthesis when the grade of operation of the prosthesis by a user is unknown. For embracing a wide number of users and at the same time to fulfil the law it is then necessary to design the prosthesis with wide and cautious safety coefficients. The use, however, of data acquisition systems on the inner tensional status of the prosthetic limb, with suitable software, can define the need of maintenance of the prosthesis, without oversizing the limb and therefore reducing the weight and the encumbrance thereof.

### Summary of the invention

It is a general feature of the present invention to provide a prosthesis for above-knee amputees that restores the walking activity of an amputee in a similar way to that of a non-disabled person both at the knee joint and at the ankle/foot joint, improving the existing techniques and solving the above described problems.

It is also a feature of the invention to provide an artificial limb that reproduces all the features of a healthy limb, and, in particular, allows detecting data on the surrounding environment, and on the relative position of the limb with respect to the surrounding environment.

It is another feature of the invention to provide an artificial limb that allows also detecting data on the inner status of the limb, in particular on the stress-strain status to which the limb is subject, allowing an analysis of the instant stiffness conditions of the joints involved in the prosthesis, i.e. those of the knee and of the ankle/foot joints.

It is a further feature of the invention to provide an artificial limb that has an improved control logics with respect to the prior art, allowing to choose desired operations for ensuring comfort and safety when walking and to mimic the gait and other postures of a non-disabled person.

It is also a feature of the invention to provide an artificial limb for dissipating/recovering/providing energy when walking concerning the knee joint and/or the ankle joint, allowing to even recover energy of first species (for example mechanical work) in dissipative phases of the gait and to use it in phases of the gait when there is demand of energy.

It is a further feature of the invention to provide an artificial limb that makes it possible to above-knee amputees to mimic a natural gait, with reduced energy consumption by the patient, with readiness of response within the gait cycle, with adaptation to a variety of types of path, to minimize the request for energy to feed to the prosthesis. Furthermore, it is a feature of the invention to provide a limb that reproduces the posture of a non-disabled person also during an activity such as riding a bicycle, sitting, etc.

It is also a feature of the invention to provide an artificial limb that assists the gait for amputees with low capacity of gait, i.e. elder people or people who have difficulty to repeat the gait.

Another feature of the invention is to provide an artificial limb that ensures dynamic damping response, ensuring comfort and stability during the gait avoiding innatural stiffening phenomena.

It is also a feature of the invention to provide an artificial limb that increases safety of control allowing achieve higher clearance in the so-called Toe-Clearance phase.

It is also a feature of the invention to provide an artificial limb that allows to control of the stiffness of the joints, to avoid shocks, to recover the position of the ankle in the presence of kerbs, to ensure a high safety of gait and to avoid to the patient to monitor continuously obstacles of the surrounding environment.

It is another feature of the invention to provide an artificial limb for changing the gait speed in a gait cycle.

It is another feature of the invention to provide an artificial limb that increases the range of the batteries of the prosthesis, with ease of charge and change of the batteries.

These and other objects are achieved by a prosthesis for lower-limb amputees, said prosthesis comprising a foot segment and a tibial segment to each other pivotally articulated, said tibial segment being articulated pivotally by an ankle to said foot segment at an ankle joint, wherein the gait cycle of said prosthesis comprises a so-called swing phase, between the disengagement of the toe of the foot and the support of the heel of the foot, and a so-called stance phase, comprising the support of the heel, the support of the sole and the disengagement of the toe,
wherein said ankle joint comprises:
- an actuator, in particular a gear motor,
- a microprocessor means that is adapted to control the movement of the ankle joint by said actuator for reproducing the natural gait cycle and defining the relative position of the foot with respect to the tibia,
- a position transducer at the articulation axis of the ankle that reproduces the movement of the tibia with respect to the foot, said position transducer measuring the rotation of the foot segment at the ankle and supplying an angular position signal;
- a program means residing in the microprocessor, said program means calculating the foot/tibia angular speed according to said position signal;
wherein said program means arranged to generate a plurality of n-dimensional curves that reproduce the gait cycle,
- and wherein said program means cause said actuator to belong to one of said curves for characterizing a predetermined gait or posture condition.

In particular, for the ankle n-dimensional curves can be defined that show the combination of variable values such as: the foot/tibia relative angle, the foot/tibia angular speed, etc. Said variables generate n-dimensional closed curves in n-dimensional spaces not crossing each other such that the fact of belonging to one of said curves is an univocal characteristic of a certain walking condition or of a certain posture (walking at a certain speed, thrust on a pedal, etc...).

In particular, said n-dimensional curves can be bi-dimensional position-angular speed curves. The curves can describe therefore the gait of the patient or typical postural status; they can be predetermined curves, based on some geometric parameters of the patient (weight, size of the foot, knee-ground height, etc.) and/or can be curves learnt by the prosthesis during the use, and exploited then as reference curves for checking the correct gait. In particular, a detachment from the curves can define gait defects, and therefore the curves ensure to the prosthesis safety conditions to avoid dangerous phenomena (such as fall).

The curves can be operated starting from base signals such as those above cited but can be suitably enlarged by addition of sensors, capable of generating more and more complex n-dimensional spaces and that define the gait status.

Advantageously, said program means starting from said n-dimensional curves that describe in a predefined way the gait of the patient, can learn new curves during the gait, said new curves being used as reference curves for checking the correct gait, in order to represent at best the gait of the patient. In other words, the curves describe the gait of the patient and the program means starting from predetermined curves learn new curves during the use, and use the new curves as reference curves for checking the correct gait, in particular the program means arranged to determine a detachment from the new curves for defining gait defects.

Advantageously, said program means can be arranged to determine a detachment from said predetermined n-dimensional curves for defining gait defects, such as walking defects, and therefore to ensure to the prosthesis to start a safety response to avoid dangerous phenomena, such as fall.

Advantageously, said program means can cause said actuator to move from a curve to another curve in a same gait cycle.

Advantageously, said ankle joint can comprise a damper that assists the actuator or replaces it in a passive phase, in particular said damper on the ankle can be of a controlled impedance type that is controlled by said program means through said curves. The damper on the ankle can assist the gait for example when the actuator is not capable alone of providing all the dissipative action necessary in phase of support.

In particular, said actuator can be arranged to work as brake motor throughout said step of support accumulating energy in a battery. In particular, the actuator carries out both the active drive of the relative position of the foot with respect to the tibia, in particular in the swing phase, maximizing the height between the foot toe and the ground, and the action as brake motor accumulating energy in a battery.

Preferably, said ankle can comprise, furthermore, a spring in series to the hinge of the ankle for reducing the engagement in term of couple of the actuator.

Advantageously, the ankle/foot in its foot segment may comprise an under set comprising a heel (heel and tarsus) a central portion (metatarsus) and a toe portion (phalanxes) that occupies the last third of the overall length of the foot, where the metatarsus and the phalanxes have preferably an elasticity that makes it possible a relative rotation, and a inner damping means is provided made by the material that defines part of the foot, in particular the foot can be obtained by means of sheets of a composite material with function of leaf springs and with sagittal discharges for conferring flexion in the front plane.

Advantageously, said prosthesis can comprise, furthermore, a femoral segment that can be fixed to a femoral fastening and pivotally connected to said tibial segment about an articulation axis that reproduces the movement of the knee, wherein said knee articulation comprises a hydraulic damper, wherein said hydraulic damper has respectively an upper fastening and a lower fastening connected respectively with said femoral segment and said tibial segment and that is arranged for damping the relative movement of said tibial segment with respect to said femoral segment, so that in a part of the gait cycle the tibial segment is braked with respect to the femoral segment. In particular, the hydraulic damper comprises:
- a cylinder-piston and a stem hinged to said piston,
- a microprocessor adjustment means for adjusting the damping reaction of said damper,
- a force transducer arranged, in particular on the stem, such that the microprocessor receives a force signal by the force transducer and operates the adjustment means for adjusting the reaction of said damper responsive to the force signal present in said damper.

Advantageously, a spring can be provided arranged in parallel to said damper, in particular said damper is arranged to work as mechanical abutment in the configuration of completely extended articulation.

In particular, an actuator can be provided, in particular a gear motor, mounted at the knee with main function of generator for assisting the damper in its function of dissipator during the step of flexion and of extension of the knee that are dissipative phases of the gait, and for recovering energy in the form of counter-electromotive force to coils of the motor, said energy being suitably loaded in a storage unit that is available also to said actuator at the ankle.

Advantageously, a position transducer can be provided at the articulation axis that reproduces the movement of the knee, said position transducer measuring the rotation of the knee, in particular inclination sensors, accelerometres, force sensors with respect to the foot toe and to the heel can be provided, said sensors and transducers supplying relative signals to a program means residing in the microprocessor, said program means generating n-dimensional curves in n-dimensional spaces that show the combination of variable values selected from the group consisting of: the femur/tibia relative angle, the relative femur/tibia angular speed, the stress along the axis of the damper, the foot/tibia relative angle, the foot/tibia angular speed, said program means causing said actuators to belong to one of said curves for characterizing a predetermined gait or posture condition.

In particular said curves can describe the gait of the patient, wherein said program means starting from predetermined curves can learn new curves during the use, and use said new curves as reference curves for checking the correct gait, in particular said program means arranged to determine a detachment from said curves for defining gait defects.

Advantageously, said force transducer on the stem can be a ring dynamometer, such as a Morehouse load cell, in particular said Morehouse load cell can comprise a resilient portion of said stem with a hole perpendicular to the axis and a plurality of strain gauges that convert a lengthening or a compression into a change of electric resistance.

Advantageously, said strain gauges can be connected to each other in a Wheatstone bridge configuration, in order to provide a signal that is adapted to be computed by an algorithm for calculating the applied force, in particular a high-pass filter is provided for checking the proper frequencies of the impact of the heel with the ground. In particular, a high-pass filter is provided for checking the proper frequencies of the impact of the heel with the ground in order to give an information with a comparation of the frequency content of the low-pass filter read by the load cell for determining the initial gait status, in particular a high-pass filter is provided so that said program means carry out a comparation of said high and low filters on the read frequency content, and create said curves in an n-dimensional environment.

Alternatively, said force transducer on the damper can be a load cell arranged at said lower fastening of said damper. This way, it is possible an instant verification of the loading condition on the damper and a feedback control on the dynamic behaviour of the knee.

Advantageously, said prosthesis can comprise a foot segment and a tibial segment to each other pivotally articulated, said tibial segment being articulated pivotally by an ankle to said foot segment at an ankle joint, wherein the gait cycle of said prosthesis comprises a so-called swing phase, between the disengagement of the foot toe and the support of the heel of the foot, and a so-called stance phase, comprising the support of the heel, the support of the sole and the disengagement of the toe, wherein said ankle joint comprises an actuator and a microprocessor means that is adapted to control the movement of the ankle joint for reproducing the natural gait cycle and defining the relative position of the foot with respect to the tibia, wherein said actuator has a motor co-axial to the tibia.

Advantageously, the knee, ankle/foot joints can provide the presence of electro/mechanical elements adapted to generate movement (active phase) or damping the movement (passive phase). In particular, the active phase can be obtained by:
- resilient springs (torsion or flexion or leaf springs or of other nature, having fixed or adjustable stiffness)
- gear motors (brushless, brushed, USM, magnetic, etc.)

In particular, the passive phase is obtained by:
- dampers (hydraulic or pneumatic or magneto-rheological or hysteresis, of axial or torsional type)
- electro/magnetic brakes with generation of suitable counter-electromotive forces. In the latter case it is desirable to detect the energy delivered - if of enough good quality voltage and amperage - In order to store it in a suitable storage unit that can be in common between the described parts, and said electro/magnetic brake turns into a generator.

Preferably, for reducing the energy consumption in the prosthesis, and for increasing the range of the batteries of the motor/generator system, variable-pitch springs can be provided that allow to achieve an ideal stiffness, i.e. low for small angles of travel between the femoral segment and the tibial segment, and high for wider angles of travel. Owing to these features, said prosthesis is adapted to amputees with low capacity of gait, i.e. elder people or insecure in the gait, thus assisting the gait.

Advantageously, the knee and the ankle can share a same storage unit; therefore when the possible motor/generator system connected to the knee has to work as motor it can use the energy accumulated in the storage unit, and said energy had been previously produced by the motor/generator system connected to the ankle in the phases where the latter had worked as generator. [and vice-versa]

Furthermore, a microprocessor can be provided that is adapted to analyse the data measured by the transducers, comparing them with the data recorded in a memory unit, for determining, among the recorded data, the family of curves and the curves more adapted to represent the current gait, so-called ideal curve.

Said microprocessor can adjust the reaction of the damper and of the motors in order to minimize the error, which can be defined, for example, as the distance, in a n-dimensional space, between the current point, whose coordinates are defined by the measurements made by the transducers in the current instant, and the corresponding point of the ideal curve, or as error of force according to the relative angle and the derivative of the angle of the articulation (knee or ankle).

Advantageously, said microprocessor can decide, according to the amount of the error, referred to the ideal curve used and the family to which the curve belongs, whether continuing to follow the current ideal curve, or using a different ideal curve or changing family of curves.

Advantageously, said microprocessor can be arranged to optimize the gait responsive to the evolution of the psychological-physical status of the patient, therefore the patient can walk always at best both in the phases immediately following the amputation, when the amputee is most insecure, and when more dexterity has been acquired. A further advantage is that the time for rehabilitation is reduced, since the patient is continuously assisted by a device that carries out the function of electronic rehabilitator because it works for correcting and improving the gait.

### Brief description of the drawings

The invention will be now shown with the following description of an exemplary embodiment thereof, exemplifying but not limitative, with reference to the attached drawings in which:
- Fig. 1 shows the links of a prosthesis for lower-limb amputees, according to the invention, showing also the joints of the prosthesis and the main angles of the joints in case of a leg/ankle prosthesis or of a prosthesis for above-knee amputees;
- Fig. 2 shows a perspective view of a leg/ankle prosthesis, according to the invention, comprising a foot segment connected to a tibial segment;
- Fig. 3 shows a cross sectional view of the leg/ankle prosthesis of Fig. 2, according to the invention, depicting the co-axial arrangement of a gear motor at the tibia that operates the relative movement of the foot and, furthermore, shows a damper;
- Fig. 4 shows a perspective elevational front view of the leg/ankle prosthesis of Fig. 1 in which the damper between foot and tibia is not present;
- Fig. 5 shows a two-dimensional position-angular speed curve that reproduces the gait cycle; this curve has been generated in the phase of support (stance) of the foot, for maximizing in the swing phase the height between the foot toe and the ground;
- Fig. 5A shows a family of two curves, determined by a position transducer located on the ankle, which reproduce the gait cycle and are variable responsive to the gait type and conditions, of increasing size responsive to the gait speed; advantageously, have been depicted only two curves even if many of them can be provided, one for each predetermined speed i.e. 2-3-4-5 Km/h and also according to geometric parameters of the patient;
- Fig. 5B shows the case of a angular speed/angle curve at a fixed gait speed in the plane similar to Fig. 5A, obtained from the angle/time curve (Fig. 5C) and angular speed/time curve (Fig. 5D);
- Fig. 5E shows a curve among many similar curves that represents, compared with the case of Fig. 5B, indicated with dashed line, the case of a sharp acceleration in the plane; Fig. 5F shows two curves among many similar curves that show respectively an fixed gait speed in the plane (similar to Fig. 5A) and a gait when rising on a staircase;
- Figs. 6 and 6A show a perspective view of a above-knee prosthesis in a full configuration that comprises, in addition to the above described leg-ankle articulation shown in Fig. 1, also a knee articulation which comprises a hydraulic damper that provides a damping between a femoral segment and a tibial segment;
- Fig. 7 and 7A show in a perspective enlarged view a hydraulic cylinder-piston damper connected between the femoral segment and the tibial segment;
- Fig. 7B shows in detail a stem belonging to the hydraulic damper of Fig. 7 and 7A, on which a force transducer is arranged;
- Fig. 8 shows a two-dimensional position-angular speed curve that reproduces the gait cycle generated by a microprocessor on the basis of a signal coming from a position transducer present in the knee; the two-dimensional curve defines a ideal gait cycle for a predetermined average walking speed, i.e. 2-4 Km/h;
- Fig. 9A shows a family of curves, determined by a position transducer located on the knee, which reproduce the gait cycle and are variable responsive to the gait type and conditions, i.e. 2-3-4-5 Km/h, and also according to geometric parameters of the patient;
- Fig. 9B shows a family of curves similar to Fig. 9A, where it is shown graphically a defect of gait consisting in the fact one of the curves is shifted from the family of predetermined curves;

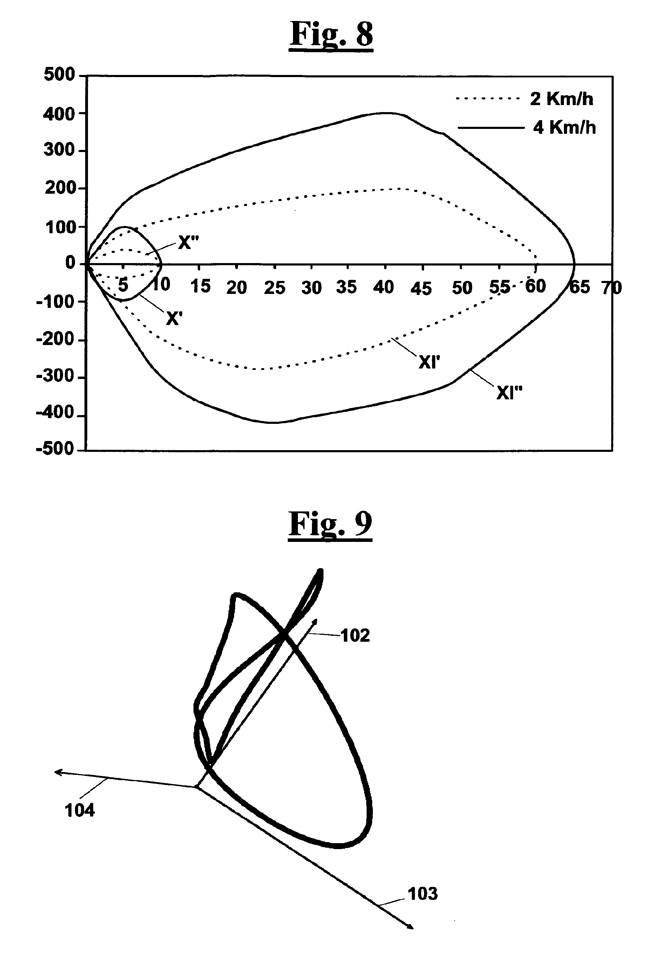

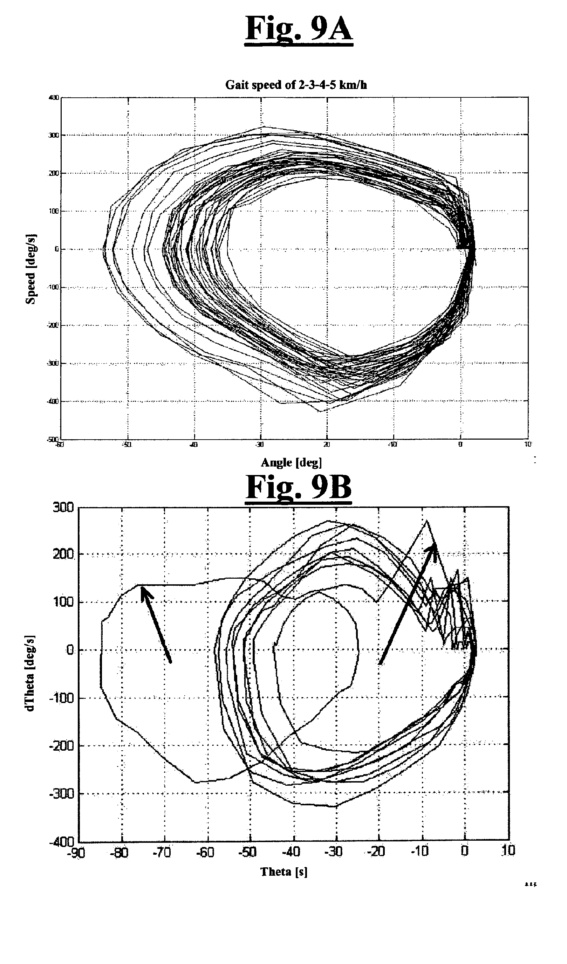

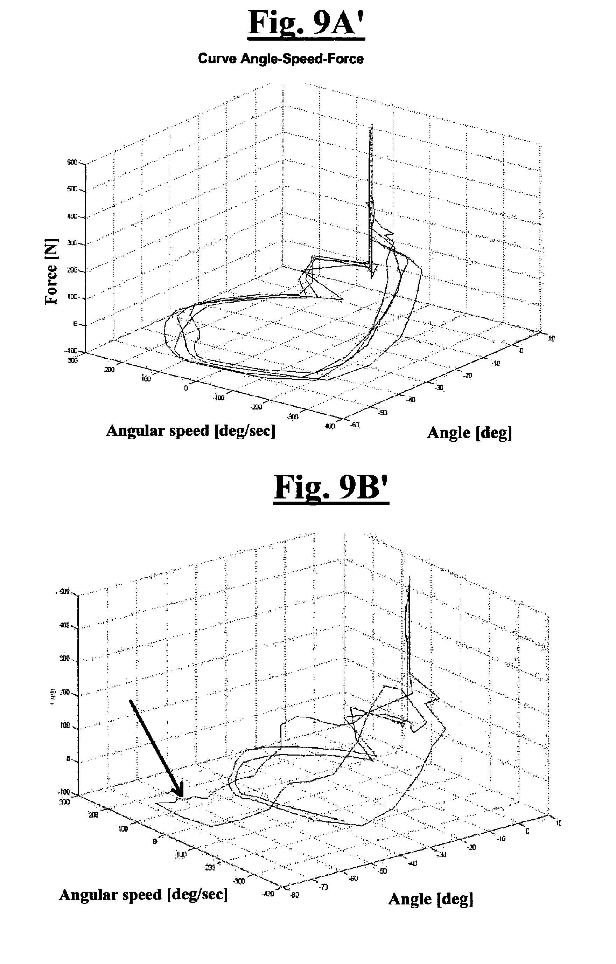

- Figs. 9, 9À and 9B' show three-dimensional curves in three-dimensional space generate with respect to the curves of Fig. 9A and 9B, with the addition of other parameters as acceleration, stress, with respect to the curves of Fig. 8;

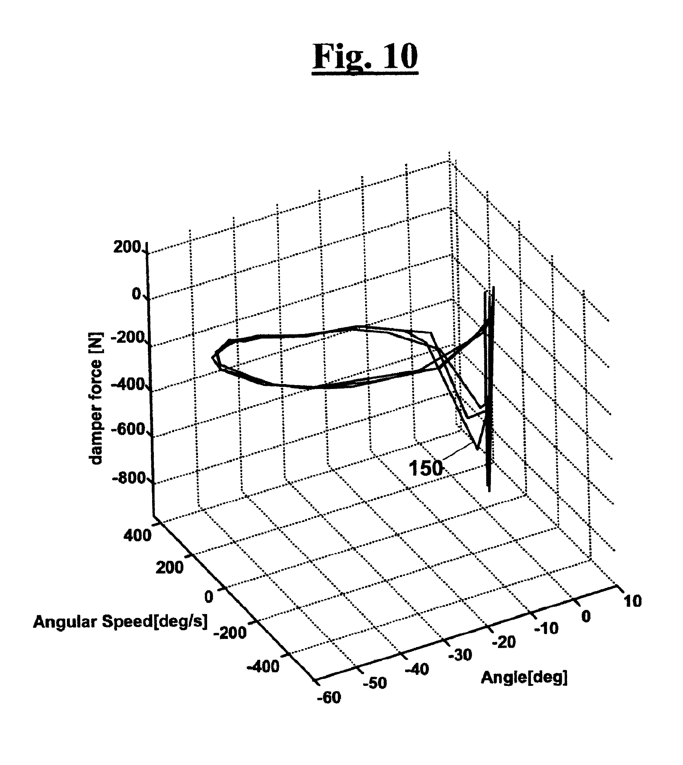

- Fig. 10 shows a family of three-dimensional curves where it is present a curve that depicts an irregular gait episode for the patient;

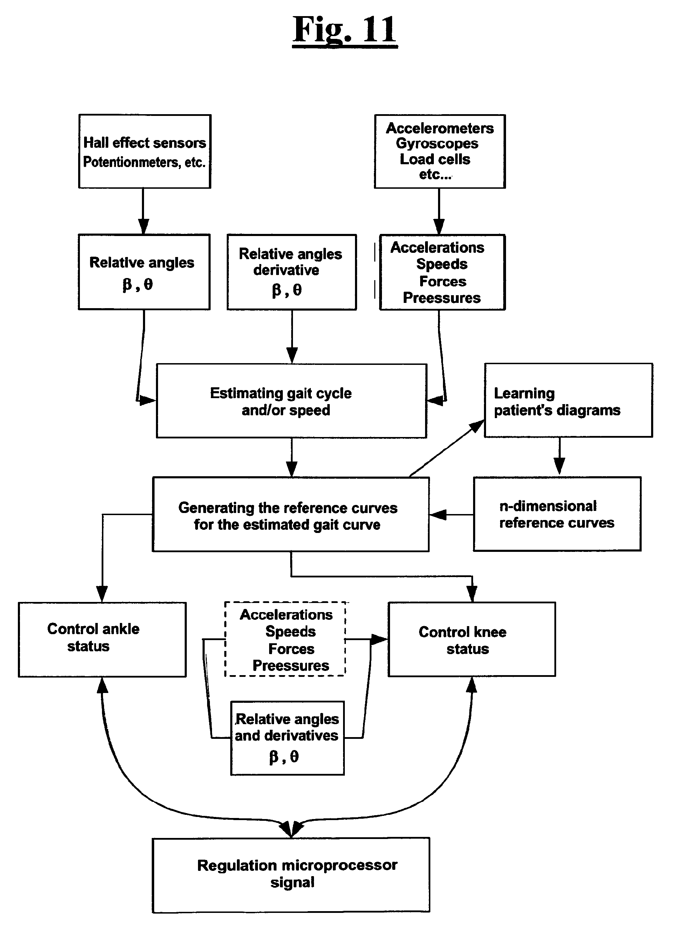

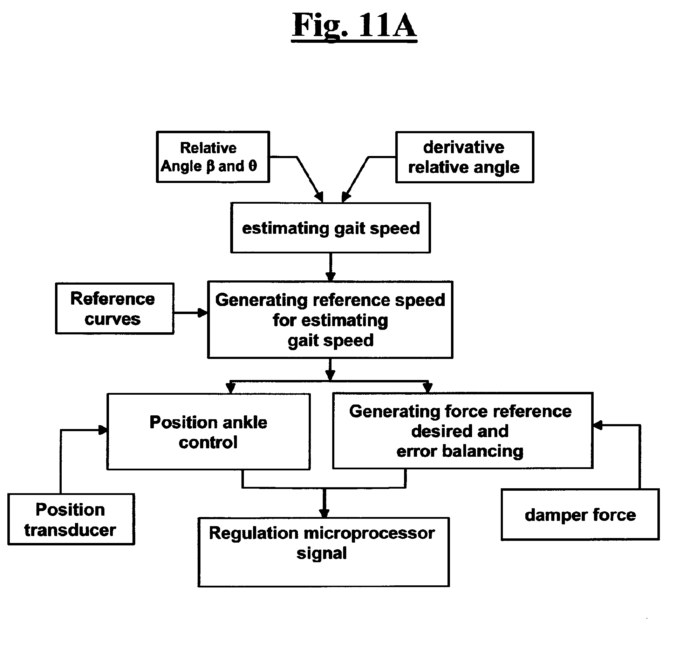

- Fig. 11 shows a block diagram of the program means for generating the above described three-dimensional curves associated with the above described parameters of acceleration, stress, etc.;
- Fig. 11A shows a particular block diagram of Fig. 11 that shows generating the curves on the basis of the position and force transducer.

### Description of a preferred exemplary embodiment

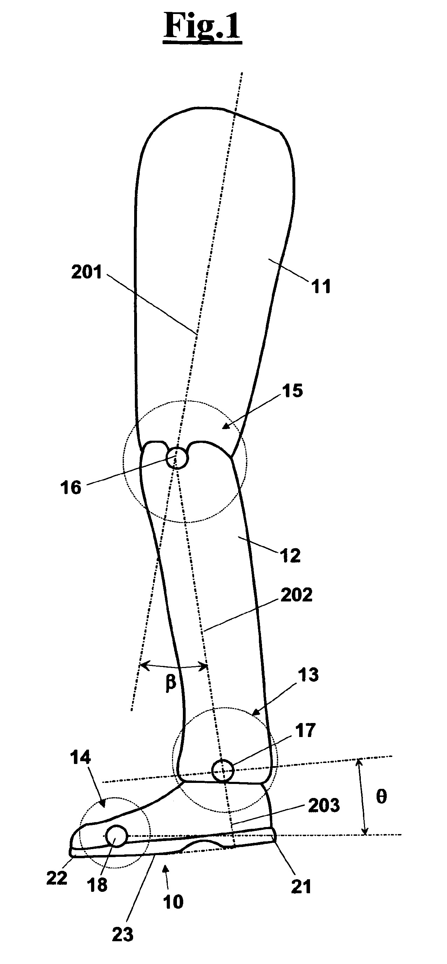

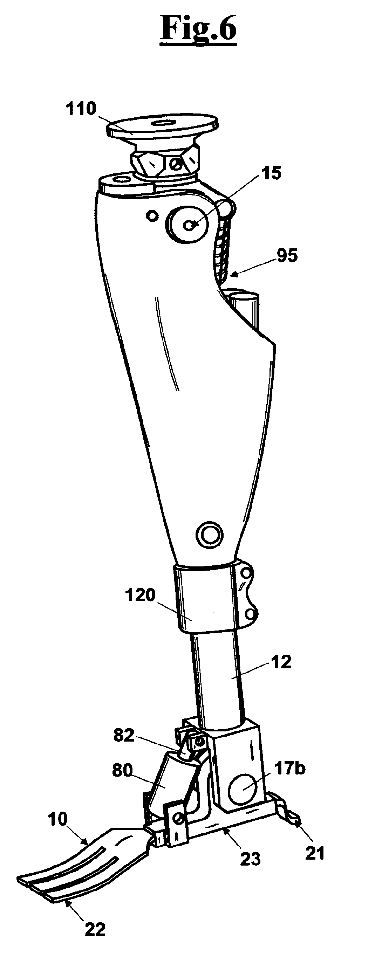

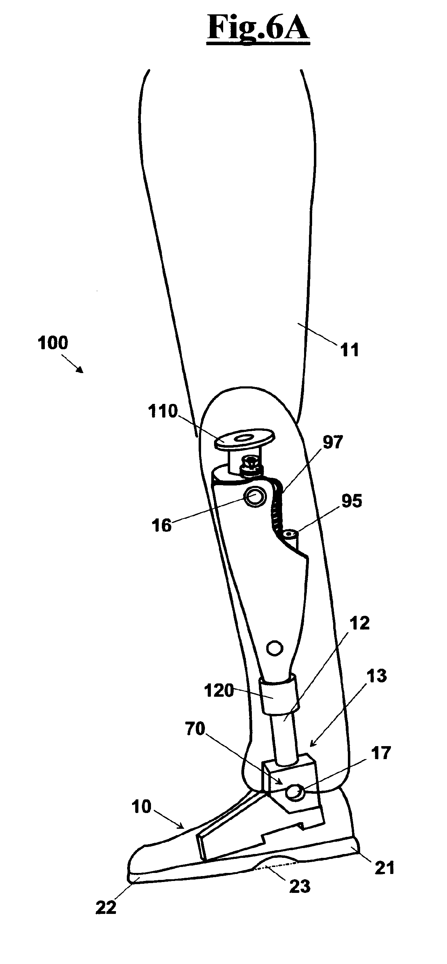

With reference to Fig. 1, a prosthesis for lower-limb amputees comprises a foot segment or foot 10 and a tibial segment or tibia 12, pivotally connected to each other that form a leg/foot prosthesis. In addition, the leg/foot prosthesis, in the exemplary embodiment for above-knee amputees, comprises a femoral segment 11 that can be fixed to a femoral fastening 110 of a patient, visible in Fig. 6, pivotally connected to tibial segment 12 about an articulation axis 16, in order to reproduce the movement of a knee joint or knee 15. In a same way, tibia 12 is pivotally articulated, at an ankle joint or ankle 13, to foot 10 by an articulation axis 17.

In Fig. 1, furthermore, sagittal axes are shown 201, 202 and 203 that define the movement relatively to femoral segment 11, to tibial segment 12 and to foot 10 and that form corresponding angles with respect to each other, of which the angle set between femur 11 and tibia 12 is indicated as β, and between tibia 12 and foot 10 is indicated as θ.

In addition, an articulation axis 18 of foot 10 defines a foot joint 14. In particular, foot segment 10 consists of a heel group 21, comprising the heel and tarsus, a central portion, comprising the sole or metatarsus 23 and a toe portion 22, made of phalanxes 22a, which occupies the last third of the overall length of foot 10.

As well known, the limb movement of prosthesis 100 comprises a so-called swing phase, between the disengagement of the foot toe 22 and the support of heel 21 of the foot, and a so-called stance phase, comprising the support of heel 21, the support of sole 23 and the disengagement of foot toe 22.

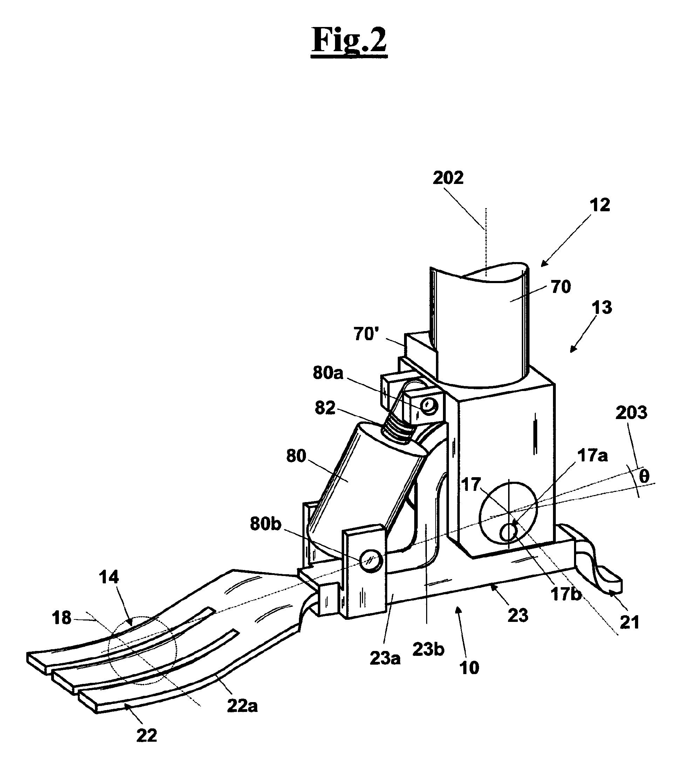

As better shown in Fig. 2, metatarsus 23 and phalanxes 22a have preferably an elasticity that makes it possible a relative rotation with respect to each other. Are, furthermore, provided means for damping inner defined by the material that is the part of foot 10 or a hinge with a damper, not shown. More precisely, foot 10 can be obtained by means of sheets of a composite material with function of leaf springs and with sagittal discharges for conferring flexion in the front plane, in order to obtain the foot joint 14 that rotates about the respective axis 18.

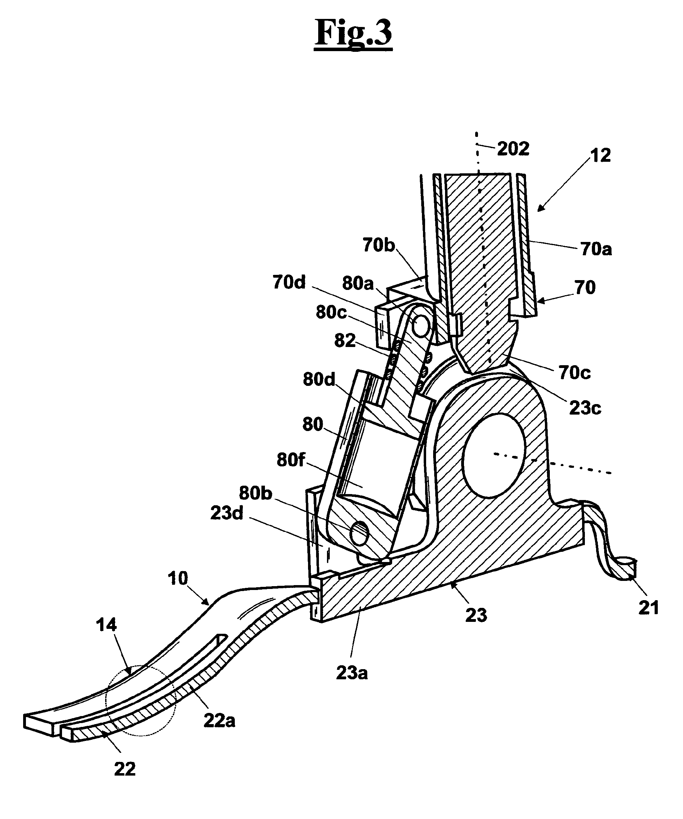

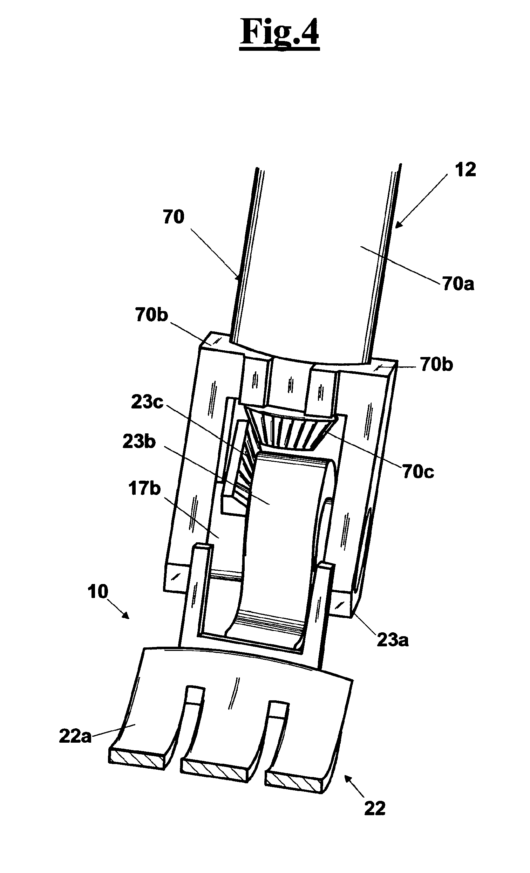

With reference to Fig. 3, a leg-foot prosthesis is shown with detail of ankle joint 13 and of foot segment 10. The lower part of tibia 12 comprises a body 70a of an actuator 70, in particular a gear motor, whose axis coincides with axis 202 of tibia 12. More precisely, from cylindrical body 70a of gear motor 70 support wings 70b extend, better shown in Fig. 4, that support a pin 17b (Fig.2) of the ankle. Furthermore, from cylindrical body 70a a pinion gear 70c with conical shape extends below. Pinion gear 70c is adapted to mesh with a toothed arch 23c present on foot 10 (Fig. 4). In particular, metatarsus 23 has a base 23a from which a hollow portion 23b extends above, in which pin 17b is housed and from which toothed arch 23c extends. This way, gear motor 70 operates a relative movement between tibia 12 and foot 10 and varies the relative angle θ (Fig. 2).

In particular, ankle joint 13 can be used in a leg-foot prosthesis or a prosthesis for above-knee amputees, comprising also a knee articulation and a femoral segment, as described below.

More in particular, gear motor 70 can be associated with a microprocessor 70' (Fig. 2) that is adapted to control the movement of ankle joint 13 for mimicking the gait. In the form shown in Fig. 3, microprocessor 70' is integrated diagrammatically in gear motor 70, but can be arranged in another zone of the prosthesis. In case of a prosthesis for above-knee amputees a single microprocessor can be provided housed at the knee, as shown hereinafter.

Microprocessor 70' (or a central microprocessor not shown and mounted at the knee that controls knee and ankle) controls then the prosthesis both in phase of raising (swing) that supporting (stance) foot 10 for defining the relative position of foot 10 with respect to tibia 12. To obtain that, microprocessor 70' communicates with a position transducer 17a arranged at articulation axis 17 (Fig.1) of ankle 13 that reproduces the movement of tibia 12 with respect to foot 10. In particular, position transducer 17a measures the rotation of ankle 13, i.e. angle θ.

In addition to the position transducer other sensors can be provided such as inclination sensors, accelerometres, force sensors arranged at foot toe 22 or at heel 21 or sensors of couple, arranged above or under ankle 13. See on this argument the block diagram of Fig. 11.

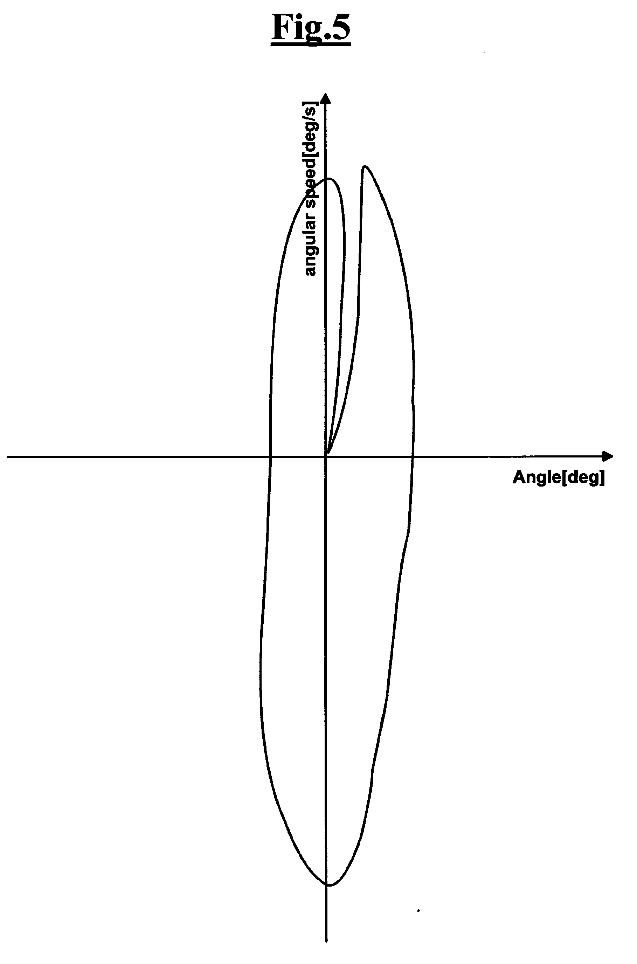

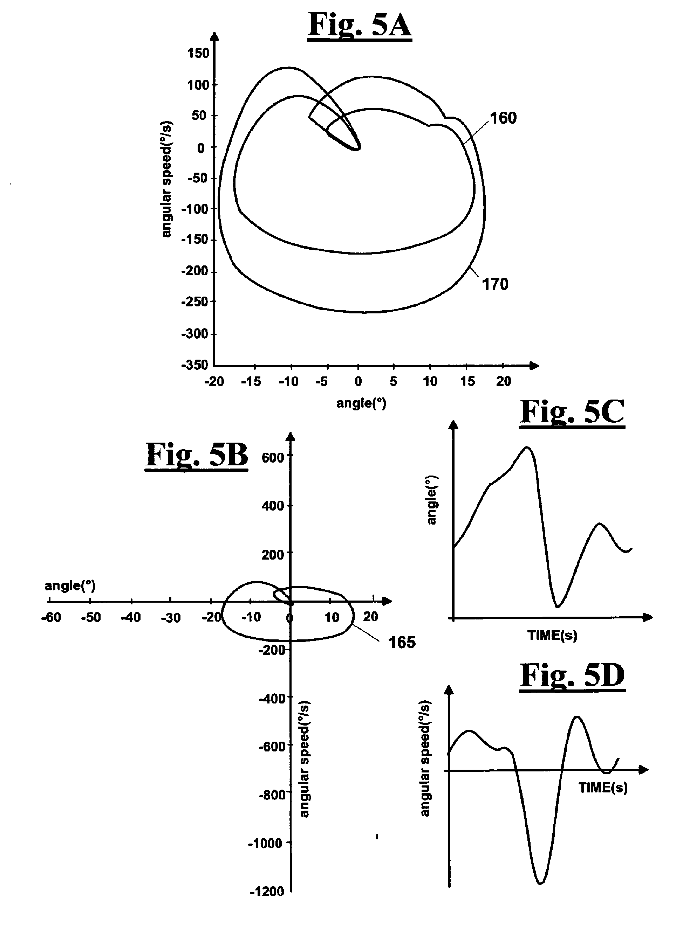

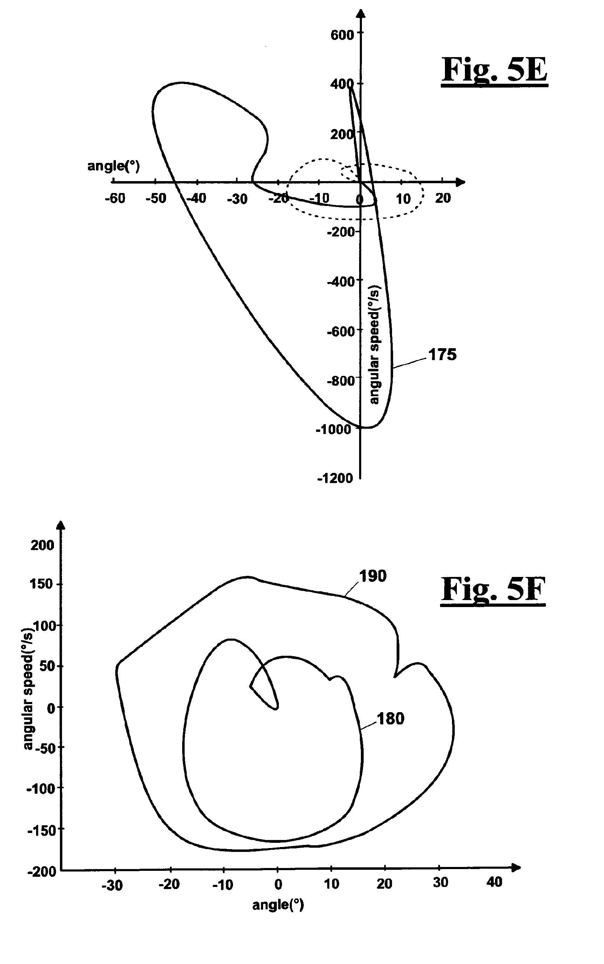

According to an exemplary embodiment of the invention, the transducer 17a provides a position signal to a program present in microprocessor 70' that calculates the relative angular speed ϑ. of foot 10 with respect to tibia 12 and generates corresponding two-dimensional position-angular speed curves that reproduce the gait cycle. A possible single curve is visible in Fig. 5. Such curve has been generated for the case where gear motor 70 administers actively the relative position of foot 10 with respect to tibia 12 at the support (stance) of foot 10, maximizing in the swing phase the height between the foot toe 22 and the ground.

Each curve is referred to a specific gait type and conditions for example at low speed, curve 160, or high speed, curve 170, for a gait on plane at constant speed (Fig. 5A). In this case the values are shown of position and speed of the ankle with respect to the tibia (reference plane perpendicular to the tibia) for a movement on a plane in the sagittal plane.

Similar curves can be generated also according to other parameters of the patient (weight, size of the foot, height knee from ground, etc...), giving rise to various possible families of curves. Then, for the ankle it is possible to define n-dimensional curves that show the combination of variable values such as: the foot/tibia relative angle, the foot/tibia angular speed, etc. Said variables generate n-dimensional closed curves in n-dimensional spaces not crossing each other such that the fact of belonging to one of said curves is an univocal characteristic of a certain walking condition or of a certain posture (walking at a certain speed, thrust on a pedal, staircase etc...).

With reference to Fig. 5B, each angle-speed curve, like this, can be obtained starting from two curves, an angle/time curve (Fig. 5C) and an angular speed/time curve (Fig. 5D).

As shown by the comparison of Fig. 5B with Fig. 5E, a walk curve 165, in the plane at constant speed, and a sprint curve 175, when the ankle is subject to a sharp acceleration, can be generated. More precisely the first curve 165 is generated by the data of previous Fig. 5A, whereas the second curve 175 relates to a running gait.

As shown in Fig. 5F, two curves among similar possible curves, respectively a curve 180 for a fixed walking speed in the plane (similar to Fig. 5A or 5B) and a curve 190 for descending from a staircase, are indicated for example. More precisely, the curve 190 is a frontal descent from a staircase, obtained for a walking speed of about 0.8sec. With reference to Figs. 5-5A, 5E, 5F, the program causes gear motor 70 to belong in position and speed to one of the generated curves for characterizing a predetermined gait or posture condition, for example, when walking in plane 180 or on a staircase 190 (Fig.5F). Providing many different curves, approximately homothetic curves, it is possible, according to the invention, to move from the conditions of a curve to the conditions of another curve even in a same gait cycle, without the need of awaiting the conclusion of a cycle for starting another at different conditions. This allows rendering the gait very similar to a natural gait, where it is possible accelerating or decelerating also in a same gait cycle.

In addition to prefixed curves that describe the gait of the patient, the program, starting from such prefixed curves, can learn new curves during the gait. Such new curves can then be used as reference curves for checking a correct gait, in order to represent at best the gait of the patient. In particular, microprocessor 70' in combination with the position sensor or other sensors generates the new curves and compares them with the respective curves already in memory, obtaining possible fault conditions.

More precisely, the program detects detachments from the above described curves and determines walking defects, i.e. defects in the gait, and then ensures to the prosthesis to work in safety and to avoid dangerous phenomena, such as fall. Then, it can learn of the types of gait, store respective curves, to use in case that such new types of gait are to be taken as model. Instead, in case of faults, their definition can be used for correcting abnormal gaits even within a same gait cycle, causing the gait parameters to belong to a corresponding correct curve.

In addition to the case of a two-dimensional curve, as above exposed, microprocessor 70' can learn n-dimensional curves (not shown) in n-dimensional spaces, with the addition of other parameters, such as acceleration, force, etc.

In addition, as shown in Figs. 2 and 3, on ankle 13 a damper 80 can be provided that assists gear motor 70 or replaces it in the passive phase. Damper 80 can be at fixed or adjustable impedance. In the latter case, it can be controlled by a program residing in microprocessor 70' that is based on the method of the two-dimensional or n-dimensional curves, as above reported.

As shown in the cross sectional view of Fig. 3, damper 80 is a cylinder-piston mechanism pivotally connected to tibia 12 and to foot 10 by means of respective hinges 80a and 80b, made on respective portions 70d and 23d of the tibia and of the foot. In particular, the connection is made between a stem 80c of damper 80, which in the relative movement between tibia 12 and foot 10 causes a damping effect during the passive phase. The position of damper 80 is an example, it could be located also in horizontal position under hinge 17.

In case of damper 80 at adjustable impedance, for example of electro-hydraulic or pneumatic or magneto-rheological or hysteresis type, it is controlled electronically and then is capable of adjusting the damping rate changing the corresponding impedance. This is calibrated according to the physical characteristics of the patient to which the prosthesis is mounted, for example weight, height, etc.

In addition, a spring can be provided 82 parallel to damper 80, in particular co-axial to the stem 80c of the damper, in order to reduce the engagement in term of couple of gear motor 70 or in case of rear flexion or plantar flexion.

In particular, when sole 23 rests on the ground, piston 80d sinks in cylinder 80f of damper 80 and spring 82 is in contrast with this movement. The use of spring 82 is preferred, but alternatively the use can be provided of a double-acting actuator. When heel 21 moves up from ground the action of spring 82 acts in parallel to gear motor 70 and follows a backward movement of stem 80c back to the starting position. Such solution ensures that in case of an abnormal operation of gear motor 70, foot 10 returns to a position which avoids phenomena of toe clearance.

Figs. 6 and 6A show an above-knee prosthesis 100 in a full configuration that comprises in addition to the above described leg-ankle articulation also the articulation 15 of the knee. In particular, knee 15 in its tibial segment 12 is pivotally connected by the "ankle" hinge 13 to the metatarsus of foot 10.

In particular, knee articulation 15 comprises a hydraulic damper 95 equipped with an upper damper fastening 110 and a lower damper fastening 120 connected respectively with femoral segment 11 and tibial segment 12. Damper 95 causes a damping of the relative movement of tibial segment 12 with respect to femoral segment 11. This way, in a part of the gait cycle tibial segment 12 is braked with respect to femoral segment 11.

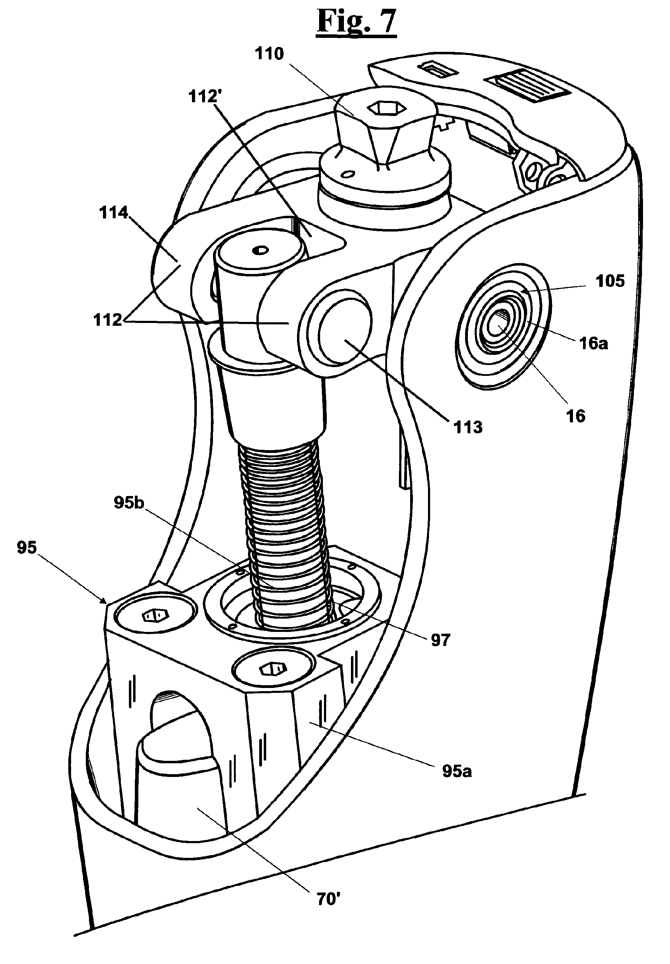

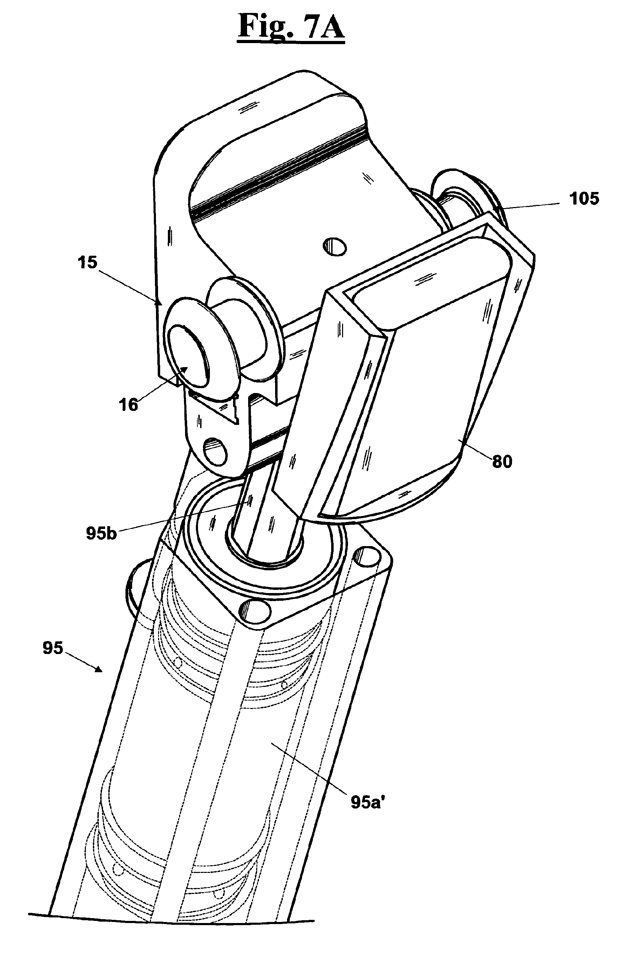

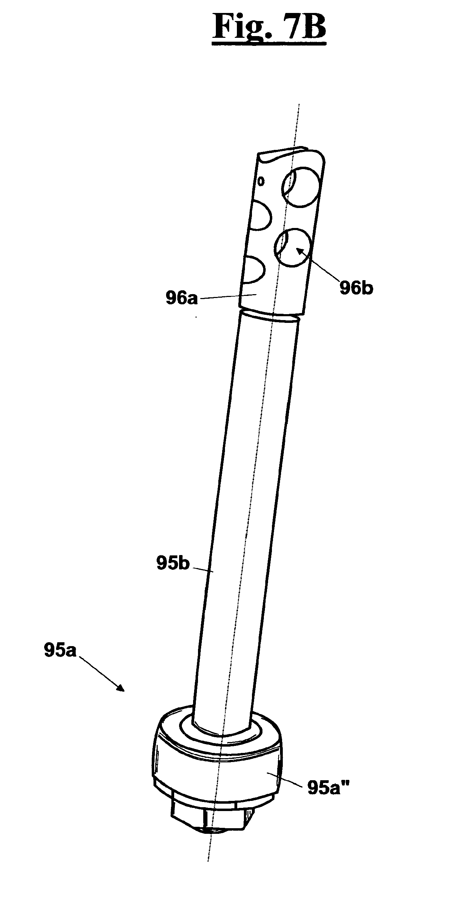

In particular, as shown in Fig. 7 and 7A, hydraulic damper 95 comprises a cylinder-piston 95a and a stem 95b hinged to the piston in addition to microprocessor 70', shown diagrammatically, for adjusting the damping reaction of damper 95. In detail, the cylinder is indicated as 95à and the piston, as shown in Fig. 7B, is indicated as 95a". In this case, microprocessor 70' that administers knee 15 is the same used for adjusting ankle 13.

In detail, stem 95b of damper 95 is pivotally connected to a portion 114 with wings 112 that define a substantially U-shaped housing 112' where it is connected stem 95b by a pin 113.

Damper 95 comprises, furthermore, a force transducer 96, shown in detail in Fig. 7B, which is arranged in particular on stem 95b of the damper, such that microprocessor 95c receives a force signal from force transducer 96 and the reaction of damper 95 changes responsive to the actual force signal.

With reference to Fig. 7, B, force transducer 96 is shown in detail i.e. a dynamometer or ring load cell, such as a Morehouse ring, put in a hole 96b of stem 95b, with axis of the hole orthogonal to the axis of the stem. The Morehouse load cell consists of a resilient metal body 96a of cylindrical shape with hole 96b perpendicular to the axis to which four strain gauges are applied that convert a lengthening or a compression into a change of electric resistance, not shown in detail. For amplifying the amount of the signal (mechanical distortion) and making it unaffected by sudden thermal changes, four strain gauges are used connected to each other in a Wheatstone bridge configuration. The electric signal obtained (differential) is about a few millivolts and requires a further amplification with a differential amplifier before being used and sent to microcontroller 70'.

The signal is then computed by an algorithm present in microprocessor 70' for calculating the force applied to transducer 96. In particular, damper 95 described above is of hydraulic type and is adapted to control high loads owing for example to shocks, ensuring a high comfort to the patient.

In particular, for checking the proper frequencies of the impact of heel 21 with the ground, in order to give an information with a comparation of the lowest frequency content read by the transducer, a high-pass filter is provided for determining the initial gait status. The use of further signals to which applying a similar technique of comparation of high-pass and low-pass filters on the read frequency content, determines generating by the limb 100 an n-dimensional environment with well defined movement curves and with the identification of "principal" events.

In other words, damper 95 is an element capable of being adjusted with continuity both in the extensive phase and in the compressive phase or only in one of the two phases. In the former case, damper 95 carries out also the function of mechanical abutment in the configuration of completely extended articulation. In this case, the lines of force that connect the mass centre of the patient with the point of support at ground of foot 10 extend through stem 95c and allow therefore to have always data on the tensional status of the limb responsive to the gait phase.

Associated to stem 95b of damper 95, furthermore, a spring 97 can be provided (Fig.7) arranged in parallel to damper 95, to assist the step of back stroke and of alignment between femur 11 and tibia 12. In particular, for reducing the energy consumption in the prosthesis, and for increasing the range of the batteries of the motor/generator system, variable-pitch springs are provided that allow to achieve an ideal stiffness, i.e. low for small angles of travel between femoral segment 11 and tibial segment 12, and high for wider angles of travel. Owing to these features, prosthesis 100 is adapted to amputees with low capacity of gait, i.e. elder people or people insecure in the gait, thus assisting the correct gait.

In addition, a gear motor 105 is provided, not shown in detail, mounted at knee 15 with main function of generator for assisting damper 95 in its function of dissipator during the step of flexion and of extension of knee 15 that are dissipative phases of the gait. In this case, it is possible to recover energy in the form of counter-electromotive force at the coils of the motor. Such energy can be suitably loaded in a storage unit 80, to feed possible further electronic systems, such as before all ankle 13 same. In an advantageous way, the same gear motor 105 can be used with function of motor to ensure an action of suitable run of tibial segment 12 with respect to femoral segment 11, thus ensuring a realignment at all the walking speeds.

From a structural viewpoint, in a preferred exemplary embodiment, knee 15 and ankle 13 share the same storage unit 80 (Fig.7B). Therefore when the possible motor/generator system connected to knee 15 has to work as motor it can use the energy accumulated in the storage unit, which was previously delivered by the motor/generator system connected to ankle 13 in the steps where the latter worked as generator, and vice-versa.

As above described, knee 15, ankle 13 and foot 14 joints are characterized by the presence of electro-mechanical elements adapted to generate movements (active phase) or damping the movements (passive phase). In particular, the active phase can be obtained by elastic springs, in particular torsion or flexion or leaf springs or of other nature, with fixed or variable stiffness, and/or gear motors, such as brushless, brushed, USM, magnetic motors, etc.

More in particular, the passive phases are obtained by dampers, for example damper 80 of ankle 13, of hydraulic or pneumatic or magneto-rheological or hysteresis type, of axial or torsional type. In addition or alternatively, by means of electro-magnetic brakes with generation of suitable counter-electromotive forces in coils. In the latter case, it is desirable to detect if the energy delivered has enough good quality voltage and amperage in order to store it in a suitable storage unit, such as battery 80 (Fig.7A), that can be shared among the described parts, and said electro-magnetic brake turns into a generator.

Normally, both in the case of the dampers and of magnetic brakes, elements of purely mechanical type can be provided, i.e. not controllable by means of a microprocessor, or elements of electromechanical type can be provided that, owing to the integration of a microprocessor, can be adjusted for dampening during the cycle of operation both in the extensive and in the compressive phases if referred to knee 15, or in the rear flexion or plantar flexion if referred to ankle 13.

As in the case of ankle 13, also in knee articulation 15 a position transducer can be provided 16a at the articulation axis 16 that reproduces the movement of knee 15. In particular, position transducer 16a measures the rotation of knee 15, i.e. angle β. As in the case of ankle 13, the transducer 16a provides a position signal to a program present in microprocessor 70' that generates corresponding two-dimensional position/angular speed curves that reproduce the gait cycle, as shown in Fig. 8.

The two-dimensional curve of Fig. 8 defines an ideal gait cycle for a predetermined average walking speed, i.e. 2-4 Km/h.

As the average speed changes, the curves change amplitude, but their shape remains unchanged.

More precisely, for a predetermined speed, an ideal curve that describes a gait is made of two subcurves, a smaller inner curve X', corresponding to the step of stance, and a larger external curve X", always corresponding in part to the step of stance, at least for its top left portion.

Both curves start from the origin. The curves change shape versus the walking speed, describing wider trajectories to increase the walking speed respectively depicted by corresponding curves XI', XI". In particular, the relative walking speed are 2 and 4 km/h, respectively for X', X" and XI', XI".

Then, since each curve defines an ideal gait cycle for a predetermined speed, and the curves change shape versus the walking speed, and each curve has a corresponding identification parameter, once detected a change of the speed in a gait cycle, it is possible to cause tibia 12 to follow a corresponding curve in that gait phase cycle, but for the new speed. This way, recognizing quickly the will of the amputees of changing the gait speed, it is possible to cause the gait to follow a curve of different amplitude with respect to that followed previously.

In addition to the simple case of two-dimensional curves, as above exposed, microprocessor 70' can generate n-dimensional curves in n-dimensional spaces, as shown in Fig. 9 with the addition of other parameters, such as acceleration or speed, obtained by accelerometres or gyroscopes, force sensors, etc.

In particular, Fig. 9 shows a curve described in a three-dimensional space that identifies univocally the gait in a plane. In the present simplified configuration, the coordinates of the space are three: tibia-femur angle rotation 102, first derivative with respect to time for the tibia-femur angle rotation 103 and force acting on the damper 104, normal to the plane containing the two axes 102 and 103. This way, it is necessary a simple identification parameter, such as the average walking speed, for discriminating a curve from the other curves of the family, as shown in Figs. 9À and 9B'.

Also in this case, the program causes the gait to follow one of the curves in position and speed for characterizing a predetermined gait or posture condition. In a same way, as described above, in addition to the predetermined curves that describe the gait of the patient, the program starting from such predetermined curves can learn new curves during the gait. Such new curves can then be used as reference curves for checking the correct gait, in order to represent at best the gait of the patient.

Furthermore, as shown in Fig. 9B', the program causes a detachment from the above described curves for defining gait defects, such as defects in the gait, and therefore to ensure the prosthesis to work in safety to avoid dangerous phenomena, such as fall.

Fig. 10 shows a further example, depicting a family of three-dimensional curves, used, in particular as reference for controlling and for adjusting the swing phase. The figure shows a curve 150 not compliant to the reference model. In this case, the reason can be a wrong gait of the patient, who may have hit against an obstacle or stumbled during the gait.

The control system present in microprocessor 70' acquires then the values of these parameters that are correlated to the will of the patient, and is capable of adjusting the behaviour of the artificial limb for ensuring a very quick response to follow the intentions of the patient substantially instantaneously. Such control system is suitable for especially for those patients that need a high dynamism. Normally it recovers, at least partially, the proprioception of the missing limb, since it binds the patient's will (for example the pressure on the fastening of the prosthesis on the skin of the stump), action and perception.

Fig. 11 shows a block diagram of a generic loop for controlling and operating the gait provided on the prosthesis. In particular, through input data such as separately or in combination, angles, speed, accelerations, forces, pressures, a gait speed is estimated. Parallelly, the program recalls from an archive the reference curves. Then, there is the generation of a reference speed obtained integrating the reference curves. This way, in a successive gait, corresponding force and error compensations are obtained through an input of the force applied by damper 95. As final step, a command is sent for adjusting damper 95.

Fig. 11A shows a flow-sheet similar to that of Fig. 11, for a loop of control and operation of the gait provided in the prosthesis for controlling the angle of the articulation and its first derivative, in order to calculate the gait speed.

To sum up, microprocessor 70' is adapted to analyse the data measured by the transducers 16a and 17a, comparing them with the data recorded in a memory unit (not shown), for determining, among the recorded data, the family of curves and the curves more adapted to represent the current gait, so-called ideal curve. Microprocessor 70' adjusts the reaction of damper 80, 95 and of motors 70, 105, in order to minimize the errors, which can be defined, for example, as the distance, in a n-dimensional space, between the current point, whose coordinates are defined by the measurements made by the transducers 16a and 17a in a current instant, and the corresponding point of the ideal curve, or as error of force, according to the relative angle and the derivative of the angle of the articulation (knee or ankle).

In particular, microprocessor 70' ascertains, according to the amount of the error, referred to the ideal curve used and the family to which the curve belongs, whether or not continuing to follow the current ideal curve, or using a different ideal curve or changing family of curves.

Such control architecture is adapted to optimize the gait responsive to the evolution of the psychological-physical status of the patient, therefore the patient can walk always at best both in the phases immediately following the amputation, when the amputee is most insecure, both when more dexterity has been acquired. A further advantage is that the time for rehabilitation is reduced, since the patient is continuously assisted by a device that carries out the function of electronic rehabilitator, because it works for correcting and improving the gait.

The foregoing description of a specific embodiment will so fully reveal the invention according to the conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

## Claims

1. A prosthesis for lower-limb amputees, said prosthesis comprising a foot segment (10) and a tibial segment (12) to each other pivotally articulated at an ankle joint (13), wherein the gait cycle of said prosthesis comprises a so-called swing phase, between the disengagement of the foot toe (22) and the support of the heel (21) of the foot, and a so-called stance phase, comprising the support of the heel (21), the support of the sole (23) and the disengagement of the toe,
wherein said ankle joint (13) comprises:
- an actuator (70), in particular a gear motor,
- a microprocessor means (70') that is adapted to control the movement of the ankle joint (13) by said actuator (70) for reproducing the natural gait cycle and defining the relative position of the foot with respect to the tibia,
- a position transducer (17a) at the articulation axis (16) of the ankle that reproduces the movement of the tibia with respect to the foot, said position transducer (17a) measuring the rotation of the foot segment (10) at the ankle and supplying an angular position signal;
- a program means residing in the microprocessor (70'), said program means calculating the foot/tibia angular speed according to said position signal;
**characterised in that**
- said program means is arranged to generate a plurality of n-dimensional curves that reproduce the gait cycle,
and that
- said program means causes said actuator (70) to belong to one of said curves for characterizing a predetermined gait or posture condition.

2. A prosthesis according to claim 1, wherein said n-dimensional curves generated by said program means show the combination of variable values selected from the group consisting of: the foot/tibia relative angle, the foot/tibia angular speed, said variables generating said closed n-dimensional curves in n-dimensional spaces not crossing each other in such a way that the fact of belonging to one of said curves is an univocal characteristic of a certain gait or posture condition.

3. A prosthesis according to claim 1, wherein said n-dimensional curves are bi-dimensional position-angular speed curves.

4. A prosthesis according to claim 1, wherein said program means starting from said n-dimensional curves that describe in a predefined way the gait of the patient, are arranged to learn new curves during the gait, said new curves arranged to be used as reference curves for checking the correct gait, in order to represent at best the gait of the patient.

5. A prosthesis according to claim 1, wherein said program means is arranged to determine a detachment from said predetermined n-dimensional curves for defining walking defects, such as defects in the gait, and therefore to ensure to the prosthesis to start a safety response to avoid dangerous phenomena, such as fall.

6. A prosthesis according to claim 1, wherein said program means cause said actuator (70) to move from a curve to another curve in a same gait cycle.

7. A prosthesis according to claim 1, wherein said ankle comprises, furthermore, a spring in series to the hinge of the ankle for reducing the engagement in term of couple of said actuator (70).

8. A prosthesis according to claim 1, wherein said actuator (70) is adapted to work as brake motor throughout said step of support accumulating energy in a battery.

9. A prosthesis according to claim 1, wherein said ankle joint (13) comprises a damper that assists said actuator (70) or replaces it in the passive phase, in particular said damper on the ankle can be of a controlled impedance type that is controlled by said program means through said curves.

10. A prosthesis according to claim 1, comprising, furthermore, a femoral segment (11) that can be fixed to a femoral fastening and pivotally connected to said tibial segment (12) about a knee joint (15), wherein said knee articulation comprises a hydraulic damper that has respectively an upper fastening and a lower fastening connected respectively with said femoral segment (11) and said tibial segment (12), and that is arranged for damping the relative movement of said tibial segment (12) with respect to said femoral segment (11), so that in a part of the gait cycle the tibial segment (12) is braked with respect to the femoral segment (11), wherein the hydraulic damper comprises a cylinder-piston and a stem hinged to said piston, and microprocessor adjustment means for adjusting the damping reaction of said damper, wherein the damper has a force transducer, in particular on the stem, and the microprocessor receives a force signal by the force transducer and operates the adjustment means for adjusting the reaction of said damper responsive to the force signal present in said damper, in particular a spring is provided arranged in parallel to said damper, in particular said damper is arranged to work as mechanical abutment in the configuration of completely extended articulation.

11. A prosthesis according to claim 5, wherein an actuator (70) is provided, in particular a gear motor, mounted on the knee joint (15) with main function of generator for assisting the damper in its function of dissipator during the step of flexion and of extension of the knee that are dissipative phases of the gait, and for recovering energy in the form of counter-electromotive force to coils of the motor, said energy loaded in a storage unit that is available also to said actuator (70) at the ankle.

12. A prosthesis according to claims 1 and 5, wherein a position transducer (17a) is provided at the articulation axis (16) that reproduces the movement of the knee, said position transducer (17a) measuring the rotation of the knee, in particular, furthermore, inclination sensors, accelerometres, force sensors can be provided with respect to the foot toe (22) and to the heel (21), said sensors and transducers supplying relative signals to a program means residing in the microprocessor, said program means generating n-dimensional curves in n-dimensional spaces that define combinations of some or all the variable obtained by the sensors present on the prosthesis, chosen for example among: femur/tibia relative angle, relative femur/tibia angular speed, stress along the axis of the damper, foot/tibia relative angle, foot/tibia angular speed, said program means causing said actuators (70) to belong to one of said curves for characterizing a predetermined gait or posture condition.

13. A prosthesis according to claims 1 and 7, wherein said curves describe the gait of the patient, wherein said program means starting from predetermined curves learn new curves during the use, and use said new curves as reference curves for checking the correct gait, in particular said program means arranged to determine a detachment from said curves for defining gait defects.

14. A prosthesis according to claim 5 wherein said force transducer on the stem is a ring dynamometer, such as a Morehouse load cell, in particular said Morehouse load cell comprising a resilient portion of said stem with a hole perpendicular to the axis and a plurality of strain gauges that convert a lengthening or a compression into a change of electric resistance, in particular said strain gauges connected to each other in a Wheatstone bridge configuration, in order to provide a signal that is adapted to be computed by an algorithm for calculating the applied force, in particular a high-pass filter is provided for checking the proper frequencies of the impact of the heel (21) with the ground.

15. A prosthesis according to claim 1, wherein said actuator (70) has a motor co-axial to the tibia.

## Patentansprüche

1. Eine Prothese für Unterschenkelamputierte, wobei die Prothese ein Fußsegment (10) und ein Tibiasegment (12) aufweist, die schwenkbar gelenkig an einem Fußgelenk (13) miteinander verbunden sind, wobei der Gangzyklus der Prothese eine so genannte Schwingphase, zwischen der Loslösung der Fußzehe (22) und der Abstützung der Ferse (21) des Fußes, und eine so genannte Haltungsphase, welche die Abstützung der Ferse (21), die Abstützung der Sohle (23) und die Loslösung der Zehe umfasst, aufweist, wobei das Fußgelenk (13) aufweist:
- einen Aktuator (70), insbesondere einen Getriebemotor,
- ein Mikroprozessormittel (70'), das eingerichtet ist, um die Bewegung des Fußgelenks (13) durch den Aktuator (70) zu steuern, um den natürlichen Gangzyklus zu reproduzieren und die relative Position des Fußes bezüglich dem Tibia zu definieren,
- einen Positionswandler bzw. -geber (17a) an der Gelenkachse (16) des Fußgelenks, der die Bewegung des Tibia bezüglich dem Fuß reproduziert, wobei der Positionsgeber (17a) die Rotation des Fußsegments (10) an dem Fußgelenk misst und ein Winkel-Positionssignal liefert,
- ein Programmmittel, das in dem Mikroprozessor (70') sitzt, wobei das Programmmittel die Fuß/Tibia-Winkelgeschwindigkeit gemäß dem Positionssignal berechnet,
**dadurch gekennzeichnet, dass**
- das Programmmittel angeordnet ist, um eine Vielzahl von n-dimensionalen Kurven zu erzeugen, welche den Gangzyklus reproduzieren, und
- das Programmmittel den Aktuator (70) veranlasst, zu einer der Kurven zu gehören, um einen vorbestimmten Gang- oder Haltungszustand zu kennzeichnen.

2. Eine Prothese gemäß Anspruch 1, wobei die n-dimensionalen Kurven, die durch das Programmmittel erzeugt werden, die Kombination von variablen Werten zeigen, welche aus der Gruppe ausgewählt sind, die besteht aus: dem Fuß/Tibia-Relativwinkel, der Fuß/Tibia-Winkelgeschwindigkeit, wobei die Variablen die geschlossenen n-dimensionalen Kurven in n-dimensionalen Räumen, welche einander nicht kreuzen, auf solche Weise erzeugen, dass die Tatsache der Zugehörigkeit zu einer der Kurven eine eindeutige Eigenschaft eines bestimmten Gang- oder Haltungszustands ist.

3. Eine Prothese gemäß Anspruch 1, wobei die n-dimensionalen Kurven bi-dimensionale Positions-Winkelgeschwindigkeitskurven sind.

4. Eine Prothese gemäß Anspruch 1, wobei das von den n-dimensionalen Kurven, welche in einer vorbestimmten Weise den Gang des Patienten beschreiben, ausgehende Programmmittel angeordnet ist, neue Kurven während des Gangs zu lernen, wobei die neuen Kurven angeordnet sind, um als Referenzkurven, zum Überprüfen des korrekten Gangs, um den Gang des Patienten am besten zu repräsentieren, verwendet zu werden.

5. Eine Prothese gemäß Anspruch 1, wobei das Programmmittel angeordnet ist, um eine Ablösung von den vorbestimmten n-dimensionalen Kurven zu bestimmen, um Gehdefekte zu definieren, beispielsweise Defekte im Gang, und um dadurch für die Prothese sicher zu stellen, dass eine Sicherheitsantwort gestartet wird, um gefährliche Phänomene wie einen Sturz zu vermeiden.

6. Eine Prothese gemäß Anspruch 1, wobei das Programmmittel den Aktuator (70) veranlasst, sich in einem selben Gangzyklus von einer Kurve zu einer anderen Kurve zu bewegen.

7. Eine Prothese gemäß Anspruch 1, wobei das Fußgelenk ferner eine Feder in Reihenanordnung zu dem Gelenk des Fußgelenks aufweist, um den Eingriff hinsichtlich einer Kopplung des Aktuators (70) zu verringern.

8. Eine Prothese gemäß Anspruch 1, wobei der Aktuator (70) eingerichtet ist, um als ein Bremsmotor während des Schritts der Unterstützung zu arbeiten, der eine Energie in einer Batterie ansammelt.

9. Eine Prothese gemäß Anspruch 1, wobei das Fußgelenk (13) einen Dämpfer aufweist, der den Aktuator (70) unterstützt oder ihn in der passiven Phase ersetzt, wobei der Dämpfer an dem Fußgelenk insbesondere einer vom gesteuerten Impedanztyp ist, der durch das Programmittel über die Kurven gesteuert wird.

10. Eine Prothese gemäß Anspruch 1, ferner mit einem Oberschenkelsegment (11), das an einer Oberschenkelbefestigung befestigt werden kann und das mit dem Tibiasegment (12) um ein Kniegelenk (15) schwenkbar verbunden ist, wobei die Knieartikulation einen hydraulischen Dämpfer aufweist, der jeweils eine obere Befestigung und eine untere Befestigung aufweist, die jeweils mit dem Oberschenkelsegment (11) und dem Tibiasegment (12) verbunden sind, und der angeordnet ist, um die Relativbewegung des Tibiasegments (12) bezüglich dem Oberschenkelsegment (11) zu dämpfen, sodass in einem Teil des Gangzyklus das Tibiasegment (12) bezüglich dem Oberschenkelsegment (11) gebremst wird, wobei der hydraulische Dämpfer einen Zylinder-Kolben und ein an dem Kolben angelenktes Hinterteil ("stern") sowie ein Mikroprozessor-Einstellmittel zum Einstellen der Dämpfungsreaktion des Dämpfers aufweist, wobei der Dämpfer einen Kraftwandler bzw. -geber, insbesondere an dem Hinterteil aufweist, und der Mikroprozessor ein Kraftsignal durch den Kraftwandler empfängt und das Einstellmittel zum Einstellen der Reaktion des Dämpfers in Reaktion auf das in dem Dämpfer vorliegende Kraftsignal betätigt, wobei insbesondere eine Feder vorgesehen ist, die parallel zu dem Dämpfer angeordnet ist, wobei insbesondere der Dämpfer angeordnet ist, um als ein mechanischer Anschlag in der Konfiguration einer vollständig ausgestreckten Artikulation zu arbeiten.

11. Eine Prothese gemäß Anspruch 5, wobei ein Aktuator (70) vorgesehen ist, insbesondere ein Getriebemotor, der an dem Kniegelenk (15) angebracht ist, mit einer Hauptfunktion eines Generators zum Unterstützen des Dämpfers in seiner Funktion als Energievernichter während des Schritts des Beugens und des Ausstreckens des Knies, welche Energievernichtungsphasen des Gangs sind, und zum Rückgewinnen von Energie in der Form von elektromotorischer Gegenkraft auf Spulen des Motors, wobei die Energie in einer Speichereinheit geladen wird, die auch für den Aktuator (70) an dem Kniegelenk verfügbar ist.

12. Eine Prothese gemäß Ansprüchen 1 und 5, wobei ein Positionswandler bzw. -geber (17a) an der Gelenkachse (16) vorgesehen ist, welcher die Bewegung des Knies reproduziert, wobei der Positionswandler (17a) die Rotation des Knies misst, wobei insbesondere außerdem Neigungssensoren, Beschleunigungsmessmittel, Kraftsensoren bezüglich dem Fußzeh (22) und der Ferse (21) vorgesehen sein können, wobei die Sensoren und Wandler Relativsignale zu einem in dem Mikroprozessor sitzenden Programmmittel liefern, wobei das Programmmittel n-dimensionale Kurven in n-dimensionalen Räumen erzeugt, welche Kombinationen von einigen oder allen durch die Sensoren an der Prothese erhaltenen Variablen definieren, die beispielsweise gewählt werden aus:
Femur/Tibia-Relativwinkel, Femur/Tibia-Relativwinkelgeschwindigkeit, Spannung beziehungsweise Belastung entlang der Achse des Dämpfers, Fuß/Tibia-Relativwinkel, Fuß/Tibia-Winkelgeschwindigkeit, wobei das Programmmittel die Aktuatoren (70) veranlasst, zu einer der Kurven zu gehören, um einen bestimmten Gang- oder Haltungszustand zu kennzeichnen.

13. Eine Prothese gemäß Ansprüchen 1 und 7, wobei die Kurven den Gang des Patienten beschreiben, wobei das Programmmittel von vorbestimmten Kurven startet und neue Kurven während des Einsatzes lernt, und die neuen Kurven als Bezugskurven verwenden, um den korrekten Gang zu überprüfen, wobei das Programmmittel insbesondere angeordnet ist, um eine Ablösung von den Kurven zu bestimmen, um Gangdefekte zu definieren.

14. Eine Prothese gemäß Anspruch 5, wobei der Kraftwandler an dem Hinterteil ein Ring-Dynamometer, beispielsweise eine Morehouse-Lastzelle, ist, wobei die Morehouse-Lastzelle insbesondere einen elastischen Abschnitt des Hinterteils mit einem Loch senkrecht zu der Achse und einer Vielzahl von Belastungs-Messeinrichtungen aufweist, die eine Ausdehnung oder eine Kompression in eine Veränderung von elektrischem Widerstand konvertieren, wobei insbesondere die Belastungs-Messeinrichtungen miteinander in einer Wheatstone-Brückenkonfiguration verbunden sind, um ein Signal zu liefern, das eingerichtet ist, um durch einen Algorithmus zum Berechnen der angewandten Kraft berechnet zu werden, wobei insbesondere ein Hochpassfilter vorgesehen ist, um die geeigneten Frequenzen des Auftreffens der Ferse (21) auf dem Boden zu überprüfen.

15. Eine Prothese gemäß Anspruch 1, wobei der Aktuator (70) einen Motor besitzt, der koaxial zu dem Tibia ist.

## Revendications

1. Une prothèse pour amputés d'un membre inférieur, ladite prothèse comprenant un segment de pied (10) et un segment tibial (12) articulés l'un avec l'autre par pivotement à une articulation de la cheville (13), dans lequel le cycle de marche de ladite prothèse comprenant une soi-disant phase aérienne, entre le dégagement de la pointe (22) et le support du talon (21) du pied, et la soi-disant phase d'appui, comprenant le support du talon (21), le support de la sole (23) et le dégagement de la pointe,
ladite articulation de la cheville (13) comprenant :
- un actionneur (70), en particulier un moteur à engrenages,
- un microprocesseur (70') adapté pour contrôler le mouvement de l'articulation (13) par ledit actionneur (70) pour la reproduction du cycle de marche naturel et la définition de la position relative du pied et du tibia,
- un transducteur de position (17a) à l'axe d'articulation (16) de la cheville, qui reproduit le mouvement du tibia relativement au pied, ledit transducteur de position (17a) mesurant la rotation du segment de pied (10) à la cheville et émettant un signal de position angulaire ;
- un programme résidant dans le microprocesseur (70'), ledit programme calculant la vitesse angulaire pied/tibia conformément audit signal de position.
**Caractérisée en ce que**
- ledit programme est conçu pour générer une série de courbes n-dimensionnelles reproduisant le cycle de marche,
et que
- ledit programme détermine l'appartenance dudit actionneur (70) à une desdites courbes pour caractériser une démarche ou une posture prédéterminée.

2. Une prothèse selon la revendication 1, lesdites courbes n-dimensionnelles produites par ledit programme indiquant la combinaison de valeurs variables sélectionnées dans un groupe composé : dudit angle relatif pied/tibia, de la vitesse angulaire pied/tibia, lesdites variables produisant lesdites courbes n-dimensionnelles fermées en espaces n-dimensionnels ne se croisant pas, de sorte que le fait d'appartenir à une desdites courbes est une caractéristique univoque d'une certaine démarche ou posture.

3. Une prothèse selon la revendication 1, lesdites courbes n-dimensionnelles étant des courbes de vitesse de position/angulaires bidimensionnelles.

4. Une prothèse selon la revendication 1, ledit programme partant desdites courbes n-dimensionnelles décrivant de façon prédéfinie la démarche du patient est conçu pour assimiler de nouvelles courbes au cours de la marche, lesdites nouvelles courbes étant configurées pour servir de courbes de référence pour le contrôle de la démarche correcte, afin de représenter au mieux la démarche du patient.

5. Une prothèse selon la revendication 1, dans laquelle ledit programme est configuré pour déterminer un détachement desdites courbes n-dimensionnelles prédéterminées pour la définition de défauts de marche, par exemple des défauts dans la démarche, et assurer ainsi que la prothèse puisse lancer une réponse de sécurité pour éviter des phénomènes dangereux, par exemple une chute.

6. Une prothèse selon la revendication 1, dans laquelle ledit programme détermine le passage dudit actionneur (70) d'une courbe à une autre courbe dans un même cycle de marche.

7. Une prothèse selon la revendication 1, dans laquelle ladite cheville comprend, en outre, un ressort en série avec la charnière de la cheville pour réduire l'engagement au niveau du couple dudit actionneur (70).

8. Une prothèse selon la revendication 1, dans laquelle ledit actionneur (70) est adapté pour servir de moteur de frein tout au long de ladite étape de support d'accumulation de l'énergie dans une batterie.

9. Une prothèse selon la revendication 1, dans laquelle ladite articulation de la cheville (13) comprend un amortisseur assistant ledit actionneur (70) ou le remplaçant dans la phase passive ; en particulier ledit amortisseur sur la cheville peut être d'un type à impédance contrôlée, contrôlé par ledit programme à travers lesdites courbes.

10. Une prothèse selon la revendication 1, comprenant en outre un segment fémoral (11) pouvant être fixé à une fixation fémorale et connecté par pivotement audit segment tibial (12) au niveau d'une articulation du genou (15), ladite articulation du genou comprenant un amortisseur hydraulique doté respectivement d'une fixation supérieure et d'une fixation inférieure reliées respectivement audit segment fémoral (11) et audit segment tibial (12), et configurées pour assurer l'amortissement du mouvement relatif dudit segment tibial (12) relativement audit segment fémoral (11), de sorte qu'au cours d'une partie du cycle de marche, le segment tibial (12) est freiné relativement au segment fémoral (11), l'amortisseur hydraulique comprenant un cylindre/piston et une tige s'articulant avec ledit piston, et le dispositif d'ajustement à microprocesseur pour l'ajustement de la réaction d'amortissement dudit amortisseur, l'amortisseur possédant un transducteur de force, en particulier sur la tige, et le microprocesseur recevant un signal de force du transducteur de force et actionnant le dispositif d'ajustement afin d'ajuster la réaction dudit amortisseur en réponse au signal de force présent dans ledit amortisseur, notamment l'installation d'un ressort monté en parallèle avec ledit amortisseur, ledit amortisseur étant notamment disposé de façon à jouer le rôle de butée mécanique dans la configuration d'une articulation entièrement déployée.

11. Une prothèse selon la revendication 5, un actionneur (70) étant prévu, en particulier moteur à engrenages, monté dans l'articulation du genou (15), dont la fonction principale est celle de générateur pour assister l'amortisseur dans son rôle de dissipateur au cours des étapes de flexion et d'extension du genou, qui sont des étapes de dissipation de la démarche, et récupérer de l'énergie sous forme de force contre-électromotrice sur les bobines du moteur, ladite énergie étant chargée dans une unité de stockage qui est disponible également pour ledit actionneur (70) à la cheville.

12. Une prothèse selon les revendications 1 et 5, dans laquelle un transducteur de position (17a) est installé à l'axe d'articulation (16) et reproduit le mouvement du genou, ledit transducteur de position (17a) mesurant la rotation du genou, en particulier, en outre, des détecteurs d'inclinaison, des accéléromètres, des détecteurs de force pouvant être fournis relativement à la pointe (22) et à la cheville (21), lesdits capteurs et transducteurs fournissant des signaux relatifs à un programme résidant dans le microprocesseur, ledit programme produisant des courbes n-dimensionnelles dans des espaces n-dimensionnels, définissant des combinaisons de certaines ou de l'intégralité des variables obtenues par les détecteurs présents sur la prothèse, et choisies par exemple parmi les suivantes : angle relatif fémur/tibia, vitesse angulaire relative fémur/tibia, contraintes le long de l'axe de l'amortisseur, angle relatif pied/tibia, vitesse angulaire pied/tibia, sous l'effet dudit programme lesdits actionneurs (70) étant affectés à une desdites courbes pour caractériser un état prédéterminé de la démarche ou de la posture.

13. Une prothèse selon les revendications 1 et 7, dans laquelle lesdites courbes décrivent la démarche du patient, ledit programme à partir de courbes caractéristiques apprenant de nouvelles courbes en cours d'usage, et utilisant lesdites nouvelles courbes en tant que courbes de référence pour le contrôle de la démarche appropriée, en particulier ledit programme étant configuré pour déterminer un écart desdites courbes pour la définition de défauts de la démarche.

14. Une prothèse selon la revendication 5, ledit transducteur de force sur la tige étant un dynamomètre annulaire, par exemple une cellule de charge Morehouse, ladite cellule de charge Morehouse comprenant notamment une partie résiliente de ladite tige avec un orifice perpendiculaire à l'axe et une série de jauges de contrainte convertissant un allongement ou une compression en variation de résistance électrique, en particulier lesdites jauges de contrainte étant reliées entre elles dans une configuration en pont de Wheatstone, de façon à émettre un signal adapté pour le calcul par un algorithme pour le calcul de la force appliquée ; en particulier un filtre passe-haut est prévu pour le contrôle des fréquences appropriées de l'impact du talon (21) sur le sol.

15. Une prothèse selon la revendication 1, dans laquelle ledit actionneur (70) possède un moteur coaxial au tibia.
